# EUROPEAN PATENT SPECIFICATION

(11) **EP 2 754 395 B1**
(45) Date of publication and mention of the grant of the patent: **12.06.2019**
(21) Application number: 14150693.1
(22) Date of filing: 10.01.2014
(51) Int. Cl.: A61B 8/08, G06T 7/00, A61B 8/00, G16H 50/20

(54) **Lesion diagnosis apparatus and method**
Läsionsdiagnosevorrichtung und Verfahren
Appareil et procédé de diagnostic de lésion

(30) Priority: 10.01.2013 KR 20130002994
(43) Date of publication of application: 16.07.2014
(73) Proprietor: Samsung Electronics Co., Ltd., Gyeonggi-do 443-742 (KR)
(72) Inventor: Lee, Jae-Cheol, Seoul (KR); Woo, Kyoung-Gu, Seoul (KR); Seong, Yeong-Kyeong, Gyeonggi-do (KR)
(74) Representative: Arnold + Siedsma

(56) References cited:
- EP-A1- 2 064 996
- WO-A2-2006/057740
- JP-A- 2009 225 904
- US-A1- 2006 274 928
- US-A1- 2011 184 291
- US-B1- 6 290 648

## Description

### BACKGROUND

### 1. Field

The following description relates to a lesion diagnosis apparatus and method.

### 2. Description of the Related Art

Computer Aided Diagnosis (CAD) is a diagnosis technique that performs a lesion diagnosis through a lesion search, feature extraction, and feature classification by analyzing medical images received from an ultrasound probe. In CAD, it is valuable to receive a medical image containing a lesion quickly and to perform a lesion diagnosis quickly and precisely. An ultrasound imaging device scans a lesion from a medical image using a probe, captures a suspected area to contain a lesion as a still image, and stores the captured image in a Picture Archiving and Communication System (PACS). A CAD apparatus receives an image stored in the PACS and performs a lesion diagnosis on the received image through lesion search, feature extraction, and feature classification. If a health care professional fails to capture an area as a still image using a probe, the CAD apparatus cannot perform a lesion diagnosis on the area, and thus, a user may fail to recognize a lesion existing in the area.

US 2006/0274928 A1 describes computer-aided detection of abnormalities in medical images and, particularly, teaches providing feedback to a user about adjusting the medical apparatus that the user is manipulating to adjust a scanning device to acquire a better image. JP 2009 225904 A teaches ultrasonic imaging comprising re-scanning of lesion candidates and automatic lesion detection. US 2011/184291 A1 teaches an ultrasonic diagnostic apparatus including an ultrasonic probe for three-dimensional scanning and configured for calculating a planned surgical line for surgical operation. US 6 290 648 B1 describes an ultrasonic diagnostic apparatus wherein when changing a scanning plane it is determined whether a previously defined point of interest is still included in the changed scanning plane.

### SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter. The invention is defined by independent claims 1, 8 and 13.

In one general aspect, a lesion diagnosis apparatus is provided, and the lesion diagnosis apparatus includes a controller configured to diagnose lesions in images received from an ultrasound probe; and a controller configured to notify a user that a lesion is detected and to provide guide information, when the lesion is detected, wherein the guide information guides the ultrasound probe toward a location of the detected lesion, and the guide information comprises at least one of a moving path, a moving distance, a moving direction, or a measured angle of the ultrasound probe.

The controller may be further configured to diagnose a non-overlapping area in a image using at least one of coordinates, a measuring range, a measured angle, or a measurement direction of the probe.

The lesion diagnosis apparatus may include a store configured to store lesion information when the lesion is detected, and the lesion information comprises at least one of features of the lesion, diagnosis of the lesion, location information of the lesion, or information about the movement of the probe after the lesion is detected.

The guide information may be provided as at least one of an audio signal, a visual signal, or a tactile signal

The controller may notify the user that a lesion is detected by generating a detection signal and the detection signal may include at least one of a visual signal, an audio signal, or a tactile signal.

In response to a determination being made that the detected lesion is different from a previously detected lesion, the controller may be configured to generate a detection signal.

The lesion diagnosis apparatus may include an input means configured to receive a confirmation from the user that the detected lesion is recognized; and the lesion information further comprises indication whether the user has recognized the detected lesion.

The controller may be configured to generate a detection signal when a lesion that has not been recognized by the user is re-detected.

The controller may be configured to generate a detection signal when a lesion is re-detected and its diagnosis is different from a previous diagnosis.

The features of the lesion may comprise at least one of shape of the lesion, boundary of the lesion, posterior acoustic shadow of the lesion, echo pattern, microlobulations, hyperechoic halo, hypoechoic nodule, duct extension, compressibility, branching pattern, or density of the lesions.

The controller may be further configured to exclude the detected lesion that has been recognized by the user, from an area to be diagnosed.

In another general aspect, a lesion diagnosis method including diagnosing, with a controller of a lesion diagnosis apparatus, lesions in images that have been received from an ultrasound probe; generating, at a controller, a detection signal to notify a user that the lesion is detected; and providing guide information to a user to move the ultrasound probe toward a location of the detected lesion, wherein the guide information guides the ultrasound probe toward a location of the lesion, and the guide information comprises at least one of a moving path, a moving distance, a moving direction, or a measured angle of the ultrasound probe.

The diagnosing of lesions may include diagnosing a non-overlapping area in an image by using at least one of coordinates, a measuring range, a measured angle, or a measurement direction of the probe.

The lesion diagnosis method may include storing lesion information when the lesion is detected, and the lesion information may include at least one of features of the lesion, diagnosis of the lesion, location information of the lesion, or information about the movement of the probe after the lesion is detected.

Providing of the guide information may comprise providing the guide information as at least one of an audio signal, a visual signal, or a tactile signal.

The detection signal may include at least one of a visual signal, an audio signal, or a tactile signal.

The generating of the detection signal may include generating a detection signal when the detected lesion is different from a previously detected lesion.

The lesion diagnosis method may include receiving a confirmation from the user that the detected lesion is recognized, and the lesion information may include indication whether the user has recognized the detected lesion.

The generating of the detection signal may include generating a detection signal when a lesion that has been detected but not recognized by the user is re-detected.

The generating of the detection signal may include generating a detection signal when a lesion is re-detected and its diagnosis is different from a previous diagnosis.

The features of the lesion may include at least one of shape of the lesion, boundary of the lesion, posterior acoustic shadow of the lesion, echo pattern, microlobulations, hyperechoic halo, hypoechoic nodule, duct extension, compressibility, branching pattern, or density of the lesions.

In another general aspect, a lesion diagnosis method including diagnosing lesions, in real-time, for images received from a probe; and generating, at a controller, a detection signal to notify a user that the lesion is detected.

The diagnosing lesions in real-time may include diagnosing lesions in a first image received from the probe; identifying a non-overlapping area between the first image and a second image received from the probe; and diagnosing lesions only in the non-overlapping area.

The non-overlapping area may be identified using at least one of coordinates, a measuring range, a measured angle, or a measurement direction of the probe.

The lesion diagnosis method may include providing guide information to guide the probe toward a location of the detected lesion; and receiving a confirmation from the user that the detected lesion is recognized.

The providing of the guide information comprises continuing to provide guide information until the user confirms that the detected lesion is recognized.

The lesion diagnosis method may include storing lesion information when the lesion is detected; and the lesion information may comprise at least one of features of the lesion, diagnosis of the lesion, location information of the lesion, information about the movement of the probe after the lesion is detected, or indication whether the user has recognized the detected lesion.

In another general aspect, a lesion diagnosis apparatus including a controller configured to receive a image captured by a probe at a first point in time, and to detect lesions in the image at a second point in time; and a controller configured to provide guide information to compensate for the movement of the probe between the first point in time and the second point in time.

The guide information may guide the probe toward a location of the lesion, and the guide information may include at least one of a moving path, a moving distance, a moving direction, or a measured angle of the probe.

The guide information may be provided as at least one of an audio signal, a visual signal, or a tactile signal.

The controller may be configured to generate an increasing volume of a sound or frequency as the user moves the probe closer to the detected lesion.

The controller may be configured to increase the intensity of vibration of the probe as the probe moves closer to the detected lesion.

Other features and aspects will be apparent from the following detailed description, the drawings, and the claims.

### BRIEF DESCRIPTION OF THE DRAWINGS

FIGS. 1 and 2 are diagrams illustrating examples of configurations of a lesion diagnosis apparatus.
FIGS. 3A to 3C are diagrams illustrating examples for explaining examples of location information of a lesion, information about movement of a probe, and an overlapping area.
FIG. 4 is a diagram illustrating an example for generating a detection signal and providing guide information.
FIG. 5 is a diagram illustrating an example of a method for generating a detection signal.
FIG. 6 is a diagram illustrating an example of a method for providing guide information.
FIG. 7 is a diagram illustrating an example of a method for generating a detection signal when a lesion is re-detected.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following description is provided to assist the reader in gaining a comprehensive understanding of the methods, apparatuses, and/or systems described herein. Accordingly, various changes, modifications, and equivalents of the methods, apparatuses, and/or systems described herein will be apparent to one of ordinary skill in the art. Also, descriptions of well-known functions and constructions may be omitted for increased clarity and conciseness.

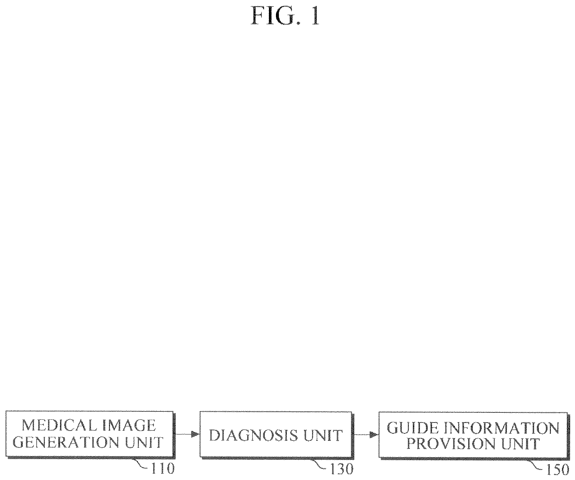

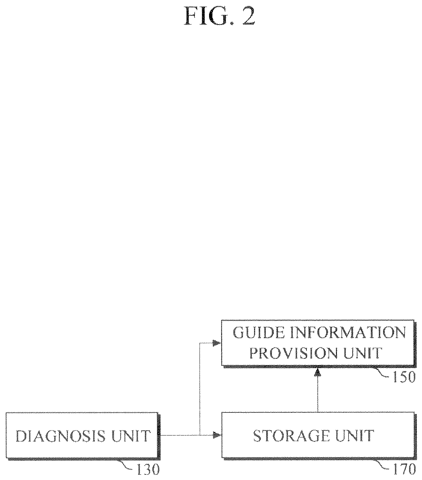

FIGS. 1 and 2 are diagrams illustrating examples of configurations of a lesion diagnosis apparatus. Referring to FIG. 1, a medical image generation unit 110 includes a probe and an image processing means. The probe transmits an ultrasound signal to a body part or tissue of a patient to obtain a medical image and receives an ultrasound echo signal reflected from the body part or tissue. The image processing means converts the ultrasound echo signal received from the probe to an electronic signal to generate a digital image. If a user moves the probe along the surface of the body part of the patient, the image processing means generates medical images in real time. As a non-exhaustive example only, the probe may include at least one of an accelerometer, a pressure sensor, or a tilt sensor (for example, a gyroscope sensor). The sensors may be installed in the inside or on the outside of the probe, and a medical image generated by the probe may be a two-dimensional (2D) image or a three-dimensional (3D) image.

A diagnosis unit 130 may diagnose lesions in medical images, which are received from the probe as the probe moves along the surface of the body part of the patient. Medical image generated by the medical image generation unit 110 is input to the diagnosis unit 130, and the diagnosis unit 130 outputs a result of a diagnosis by performing lesion detection, lesion feature extraction, and lesion classification. The diagnosis unit 130 makes a lesion diagnosis on each frame of a received medical image or on every predetermined number of frames that are sampled. As a non-exhaustive example only, one or more diagnosis unit 130 may be provided and the medical images generated by the medical image generation unit 110 may be distributed to the multiple diagnosis units 130 to be diagnosed concurrently.

If the diagnosis unit 130 detects a lesion, a guide information providing unit 150 may generate a detection signal to inform a user that the lesion is detected. As a non-exhaustive example only, the detection signal may be generated as a signal that a user is able to recognize by hearing, such as a sound or voice with a specific pattern. As another non-exhaustive example only, the detection signal may be a signal that a user is able to recognize by touching, such as probe vibration. As another non-exhaustive example only, the detection signal may be displayed in a display device (not illustrated) as a message notifying the user that the lesion is detected or may be a visual signal that a user is able to recognize, such as a turning on and off of an additionally-installed light (not illustrated). As another non-exhaustive example only, the detection signal may be generated in the form of Picture in Picture (PIP). For example, the display device (not illustrated) may be displaying a medical image received from the medical image generation unit 110 in real time and an image with the detected lesion may be displayed on one side of the displayed medical image as a Picture in Picture (PIP). The examples of the detection signal generated by the guide information providing unit 150 are only non-exhaustive illustrations of the detection signal, and other forms of detection signal are considered to be well within the scope of the present disclosure.

Referring to FIG. 2, the lesion diagnosis apparatus may further include a storage unit 170. When the diagnosis unit 130 detects a lesion, the storage unit 170 may store lesion information including, but not limited to, at least one of features of the lesion, a diagnosis of the lesion, location information of the lesion, or information about movement of a probe after the lesion is detected. The features of the lesion may include various features necessary to identify the lesion from a medical image and make a diagnosis of the lesion, such as, for example, shape, boundary, posterior acoustic shadow, echo pattern, microlobulations, hyperechoic halo, hypoechoic nodule, duct extension, compressibility, branching pattern, and density of the lesions. The diagnosis may include whether the detected lesion is malignant or benign.

The location information of the lesion may include, but is not limited to, at least one of coordinates of a detected location of the lesion, a measured angle of the lesion, or a measured direction of the lesion. The information about movement of a probe may include, but is not limited to, at least one of a moving path, a moving distance, a moving angle, or a moving direction of the probe after the lesion is detected. The lesion location information and the information about movement of the probe may be measured using sensors, such as, for example, a tilt sensor and an accelerometer.

The lesion diagnosis apparatus may further include an input means (not illustrated) to receive a user's confirmation that the detected lesion is recognized. As a non-exhaustive example only, the input means may be a mouse, a keyboard, or an additional button installed in the probe. As another non-exhaustive example only, the input means (not illustrated) may be combined with a display device. The display device can encompass any combination of display region, gesture capture region, a touch sensitive display, and/or a configurable area. The display device can be embedded in the lesion diagnosis apparatus or may be an external peripheral device that may be attached and detached from the lesion diagnosis apparatus. The display device may be a single-screen or a multi-screen display. A single physical screen can include multiple displays that are managed as separate logical displays permitting different content to be displayed on separate displays although part of the same physical screen.

As another non-exhaustive example only, the probe may serve as the input means (not illustrated). A user may, for example, confirm that the detected lesion is recognized by moving the probe in a predetermined pattern. For example, the user may not move the probe for a predetermined length of time after detecting the lesion.

If a user confirms that the detected lesion is recognized, the storage unit 170 stores the user's confirmation. In one non-exhaustive example, based on the lesion information stored in the storage unit 170, the diagnosis unit 130 may exclude a lesion, which has been recognized by a user, from an area to be diagnosed. The diagnosis unit 130 may reduce the total computation required by not diagnosing an area where the lesion has been recognized by the user. The diagnosis unit 130 may reduce the amount of total computation based on one or more of a location of the lesion, information about movement of a probe, and a measuring range of the probe.

The guide information providing unit 150 may generate guide information to guide the probe from the present location toward a location of the detected lesion. If the lesion has been detected, but not recognized by the user, the user may move a probe away from the location of a detected lesion during a processing time of the diagnosis unit 130. The user may also move a probe away from the location of a detected lesion even where the lesion has been previously detected and recognized by the user. To enable a user to move the probe toward a location of a detected lesion, the guide information providing unit 150 generates guide information based on lesion information stored in the storage unit 170 and provides the guide information to the user.

The guide information may include at least one of a visual signal, an audio signal, or a tactile signal. For example, the guide information providing unit 150 may provide a visualized image for at least one of a moving path, a moving distance, a measured angle, or a measurement direction of the probe using the lesion location information or the information about movement of the probe, which are stored in the storage unit 170. In another non-exhaustive example, if a user moves the probe toward the detected lesion, the guide information providing unit 150 may guide the probe toward the detected lesion by generating a specific sound, such as, for example, an alarm, a voice, or an increasing volume of a sound or frequency as the probe moves closer to the detected lesion. In another non-exhaustive example, if the user moves the probe in a direction toward the detected lesion, the guide information providing unit 150 may guide the probe toward the detected lesion by generating a vibration or increasing the intensity of the vibration as the probe moves closer to the detected lesion. The guide information described above are only non-exhaustive illustrations, and other types of guide information are considered to be well within the scope of the present disclosure.

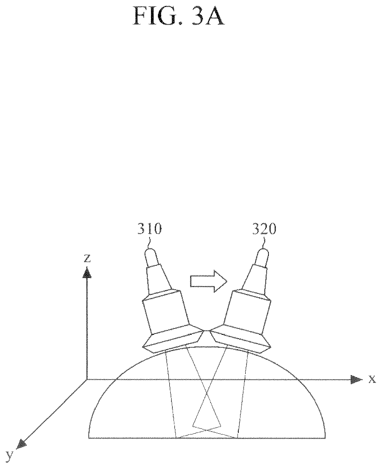

FIGS. 3A to 3C are diagrams illustrating examples for explaining location information of a lesion, information about movement of a probe and an overlapping area of an image. Referring to FIGS. 3A to 3C, when a probe is disposed at a location 310, a measurement direction ϕ, a measured angle θ, and coordinates (x, y, z) may be obtained using an accelerometer, such as, for example a gyroscope. If a user moves the probe toward a location 320, a measurement direction ϕ', a measured angle θ' and coordinates (x', y', z') may be obtained. Here, the measurement angle may be a predetermined reference angle, and the coordinates may be absolute values measured with reference to an origin of absolute coordinates. The measurement angle may also be a relative angle measured in relation to a previous measurement angle, and the coordinates may be relative values measured in relation to previous coordinates. A relative distance (d) or a relative movement direction between the two measured locations may also be obtained using the measurement angle and the coordinates. If the probe includes an accelerometer, a moving path of the probe may also be tracked.

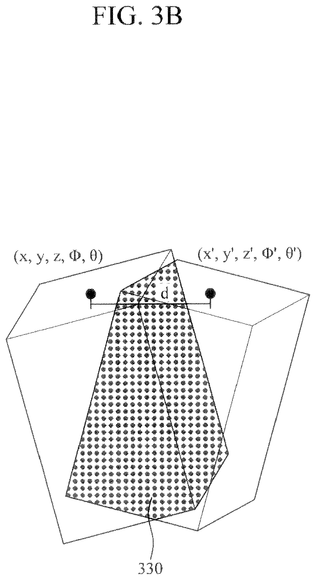

Referring to FIG. 3B, based on a measured direction, a measured angle, coordinates, and a measuring range of the probe at each of the locations 310 and 320, an overlapping area 330, which is measured more than once, may be calculated.

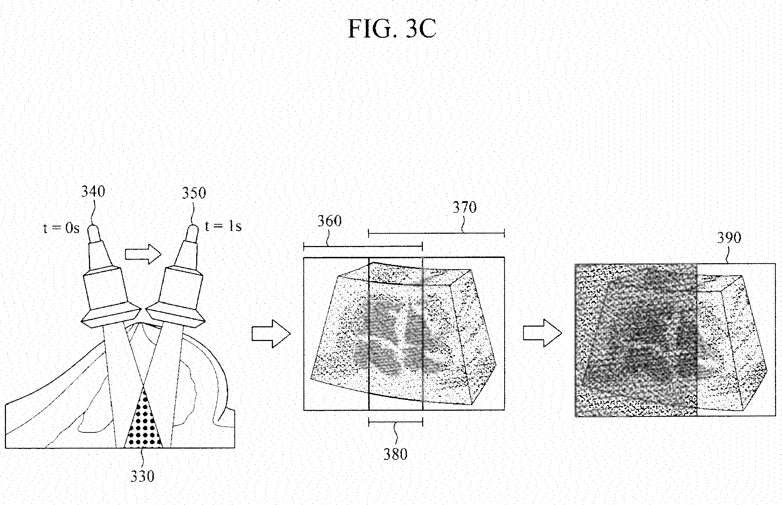

Referring to FIG. 3C, when diagnosing ailments, such as, for example, breast cancer or a thyroid gland, the diagnosis is performed by changing a measured angle and a measurement direction of a probe while moving the probe in a narrow range, and thus an overlapping area 330 is measured more than once. With reference to FIG. 3C, at time (t=0s), an image 360 is created at measurement location 340, and at time (t=1s), an image 370 is created at measurement location 350. As shown in FIG. 3C, the measurement range, measured angle, and measurement direction are different for images 360 and 370 and an overlapping area 380 is created between images 360 and 370. In this example, the total computation increases because the diagnosis unit 130 needs to diagnose the lesion more than once in the overlapping area 380. To decrease the amount of computation required and to increase responsiveness, the diagnosis unit 130 may diagnose only a new area 390 in image 370, and not repeat the diagnosis for the overlapping area 380, which is contained in both the image 370 and the previously in put image 360.

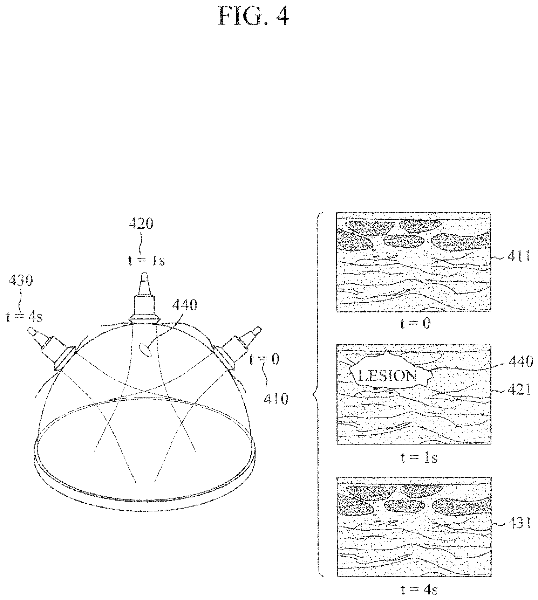

FIG. 4 is a diagram illustrating an example of generating a detection signal and guide information. Referring to FIG. 4, when diagnosing lesions in ultrasound medical images, a user recognizes a lesion by checking medical images generated in real time by a probe. The user may not recognize lesions if the user moves a probe too quickly, or if the user fails to continually concentrate on the ultrasound medical images, or if the user is not skilled at analyzing ultrasound medical images. In FIG. 4, a probe moves from measurement location 410 (at time t=0s) to measurement location 420 (at time t=1s) and to measurement location 430 (at time t=4s). A lesion 440 is not detected from an ultrasound image 411 measured at a measurement location 410, but the lesion 440 is detected from an ultrasound image 421 measured at a location 420. If the user continues to move the probe without realizing that the lesion is detected, the user will fail to recognize the lesion because the lesion will not be detected from an ultrasound image 431 measured at a location 430. If the diagnosis unit 130 detects a lesion, a guide information providing unit 150 may instantly generate a detection signal to notify the user that a lesion is detected.

In a non-exhaustive example, the guide information providing unit 150 may continue to generate a detection signal until it receives a confirmation from the user that the detected lesion has been recognized. If the user continues to move a probe without recognizing the detection signal, the guide information providing unit 150 may continue to generate the detection signal until it receives a confirmation from a user through an input means (not illustrated) that the detected lesion is recognized.

In another non-exhaustive example, if a determination is made, based on lesion information stored in a storage unit 170, that a detected lesion is different from a previously detected lesion, the guide information providing unit 150 may generate a detection signal. On the other hand, if a determination is made, based on one or more of location information of the lesion stored in the storage unit 170, information about movement of a probe, or features of the lesion, that a detected lesion is identical to a previous detected lesion, the guide information providing unit 150 may not generate a detection signal.

In another non-exhaustive example, if a lesion is detected, a user may input a confirmation through an input means (not illustrated) that the detected lesion is recognized, and the storage unit 170 may store that the detected legion has been recognized by the user. Thereafter, if a determination is made, based on one or more of location information of a lesion stored in the storage unit 170, information about movement of a probe, features of the lesion, or whether the lesion has been recognized by the user, that a lesion, which has not been recognized by a user is re-detected, the guide information providing unit 150 may generate a detection signal. In another non-exhaustive example, if it is determined that a lesion is re-detected, which was previously recognized by the user but now has a different diagnosis, the guide information providing unit 150 may generate a detection signal.

A location of a lesion may change due to pressure, and the probe may include a pressure sensor to identify the lesion with a predetermined position margin of a location of a detected lesion.

Even if a user recognizes a detection signal, the probe may leave a location of the detected lesion since there is a difference between a point in time when a medical image is captured and a point in time when the diagnosis unit 130 diagnoses the medical image, and generates the detection signal. The guide information providing unit 150 may generate guide information to guide the probe from the present location, say 430, toward a location 420 of the detected lesion.

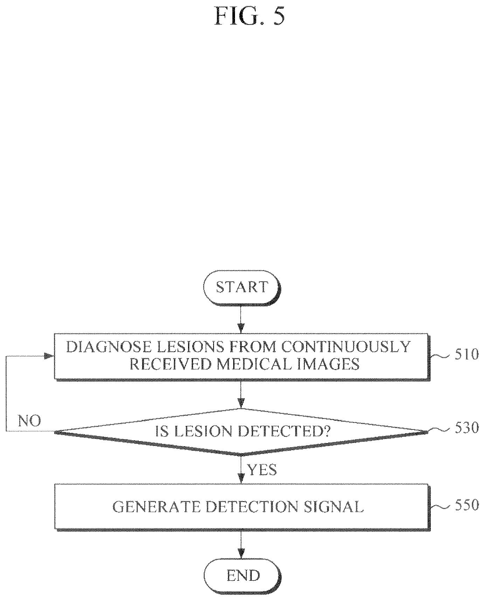

FIG. 5 is a diagram illustrating an example of a method for generating a detection signal. The operations in FIG. 5 may be performed in the sequence and manner as shown, although the order of some operations may be changed or some of the operations omitted without departing from the spirit and scope of the illustrative examples described. Many of the operations shown in FIG. 5 may be performed in parallel or concurrently. The description of FIGS. 1-4 is also applicable to FIG. 5, and thus will not be repeated here.

Referring to FIG. 5, in 510, a diagnosis unit 130 diagnoses lesions in medical images continuously received from a probe. The diagnosis unit 130 may perform a lesion diagnosis on each frame of a received medical image or on every predetermined number of frames by performing sampling of all the frames of the received medical image. One or more diagnosis unit 130 may be provided, and the medical images generated by a medical image generation unit 110 may be distributed to the multiple diagnosis units 130 to be diagnosed in parallel. In another non-exhaustive example, the diagnosis unit 130 may diagnose a non-overlapping area of the received medical images, thereby reducing the total amount of computation and increasing responsiveness.

In 530, if the diagnosis unit 130 detects a lesion, the guide information providing unit 150 generates a detection signal in 550. In another non-exhaustive example, if a user confirms that the detected lesion is recognized, the storage unit 170 may store whether the user has recognized the detected lesion.

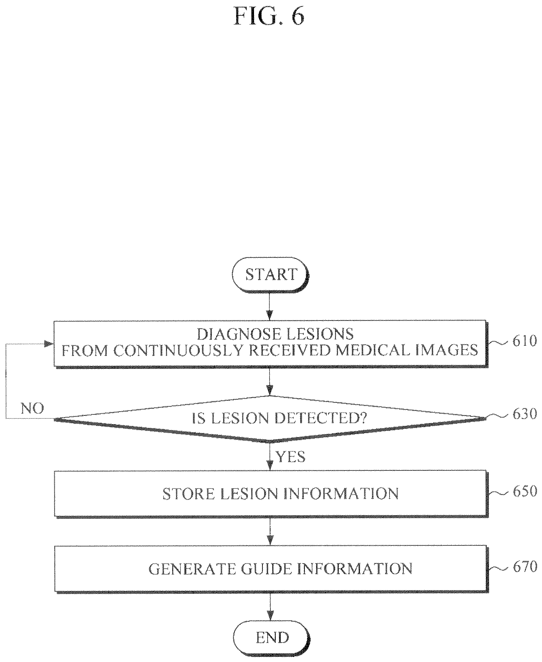

FIG. 6 is a diagram illustrating an example of a method for generating guide information. The operations in FIG. 6 may be performed in the sequence and manner as shown, although the order of some operations may be changed or some of the operations omitted without departing from the spirit and scope of the illustrative examples described. Many of the operations shown in FIG. 6 may be performed in parallel or concurrently. The description of FIGS. 1-5 is also applicable to FIG. 6, and thus will not be repeated here.

Referring to FIG. 6, in 610, a diagnosis unit 130 diagnoses lesions in medical images continuously received from a probe. In 630, if the diagnosis unit 130 detects a lesion, a storage unit 170 stores lesion information of the detected lesion in 650. The lesion information may include at least one of features of the lesion, diagnosis of the lesion, location information of the lesion, or information about movement of a probe after the lesion is detected. In 670, based on the stored lesion information, the guide information providing unit 150 may generate guide information to guide the probe from a present location toward a location of the detected lesion in 670.

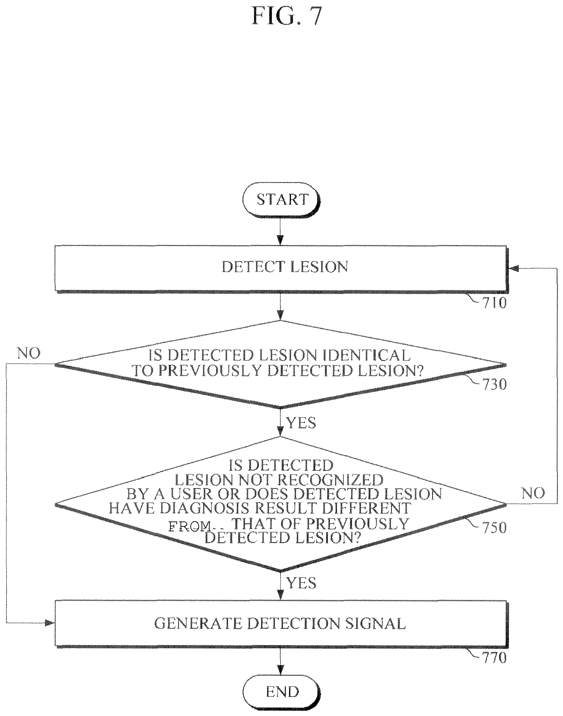

FIG. 7 is a diagram illustrating an example of a method for generating a detection signal when a lesion is detected again. The operations in FIG. 7 may be performed in the sequence and manner as shown, although the order of some operations may be changed or some of the operations omitted without departing from the spirit and scope of the illustrative examples described. Many of the operations shown in FIG. 7 may be performed in parallel or concurrently. The description of FIGS. 1-6 is also applicable to FIG. 7, and thus will not be repeated here.

Referring to FIG. 7, in 710, if a diagnosis unit 130 detects a lesion, in 730, a guide information providing unit 150 determines whether the detected lesion is identical to a previously detected lesion. In 730, based on the lesion information stored in the storage unit 170, the guide information providing unit 150 determines whether the detected lesion is identical to the previously detected lesion. In 770, if it is determined that the detected lesion is different from the previously detected lesion, the guide information providing unit 150 generates a detection signal.

In 750, it is determined whether the detected lesion is identical to the previously detected lesion but has not been recognized by the user, or if the detected lesion is identical to the previously detected lesion but its diagnosis is different from that of the previously detected lesion. If the user has not previously recognized the detected lesion or its diagnosis is different, then in 770, the guide information providing unit 150 generates a detection signal.

The lesion diagnosis apparatus, described above, notifies a user of a lesion being detected by analyzing a medical image, and provides a user with a location of the detected lesion, thereby reducing the possibility of overlooking any lesion and improving the user's convenience. In addition, the lesion diagnosis apparatus diagnosis lesions more rapidly by removing redundancies and minimizing the possibility of overlooking the diagnosis of a lesion.

The methods described above can be written as a computer program, a piece of code, an instruction, or some combination thereof, for independently or collectively instructing or configuring the processing device to operate as desired. Software and data may be embodied permanently or temporarily in any type of machine, component, physical or virtual equipment, computer storage medium or device that is capable of providing instructions or data to or being interpreted by the processing device. The software also may be distributed over network coupled computer systems so that the software is stored and executed in a distributed fashion. In particular, the software and data may be stored by one or more non-transitory computer readable recording mediums. The non-transitory computer readable recording medium may include any data storage device that can store data that can be thereafter read by a computer system or processing device. Examples of the non-transitory computer readable recording medium include read-only memory (ROM), random-access memory (RAM), Compact Disc Read-only Memory (CD-ROMs), magnetic tapes, USBs, floppy disks, hard disks, optical recording media (e.g., CD-ROMs, or DVDs), and PC interfaces (e.g., PCI, PCI-express, WiFi, etc.). In addition, functional programs, codes, and code segments for accomplishing the example disclosed herein can be construed by programmers skilled in the art based on the flow diagrams and block diagrams of the figures and their corresponding descriptions as provided herein.

The apparatuses and units described herein may be implemented using hardware components. The hardware components may include, for example, controllers, sensors, processors, generators, drivers, and other equivalent electronic components. The hardware components may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The hardware components may run an operating system (OS) and one or more software applications that run on the OS. The hardware components also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a hardware component may include multiple processors or a processor and a controller. In addition, different processing configurations are possible, such a parallel processors.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A lesion diagnosis apparatus comprising:
a memory configured to store instructions; and
at least one processor that, upon executing the stored instructions, is configured to:
detect at least one lesion in images received from an ultrasound probe movable on the surface of a body part of a patient;
display the images received from the ultrasound probe; and
notify a user that the at least one lesion is detected,
**CHARACTERIZED IN THAT**
the at least one processor is further configured to:
control the memory to store location information of the at least one detected lesion;
display guide information based on the stored location information of the at least one detected lesion, when the lesion has been detected and in response to a movement of the ultrasound probe leaving a location of the detected lesion, wherein the guide information is a visualized image for guiding the user to move the ultrasound probe toward the location the detected lesion, and the guide information comprises at least one of a moving path, a moving distance, a moving direction, or a measured angle of the ultrasound probe.

2. The lesion diagnosis apparatus of claim 1, wherein the at least one processor is further configured to:
detect at least one lesion in a first image received from the ultrasound probe;
identify a non-overlapping area between the first image and a second image received from the ultrasound probe; and
detect at least one lesion only in the non-overlapping area of the second image.

3. The lesion diagnosis apparatus of claim 1 or 2, wherein the memory is configured to store lesion information when the lesion is detected, and the lesion information comprises at least one of features of the lesion, diagnosis of the lesion, location information of the lesion, or information about the movement of the ultrasound probe after the lesion is detected; and
wherein the features of the lesion comprise at least one of shape of the lesion, boundary of the lesion, posterior acoustic shadow of the lesion, echo pattern, microlobulations, hyperechoic halo, hypoechoic nodule, duct extension, compressibility, branching pattern, or density of the lesions.

4. The lesion diagnosis apparatus of claim 3, further comprising:
an input means configured to receive a confirmation from the user that the detected lesion is recognized; and
the lesion information further comprises indication whether the user has recognized the detected lesion.

5. The lesion diagnosis apparatus of claim 4, wherein the at least one processor is configured to generate a detection signal when at least one lesion that has not been recognized by the user is re-detected.

6. The lesion diagnosis apparatus of claim 4, wherein the at least one processor is configured to generate a detection signal when at least one lesion is re-detected and its diagnosis is different from a previous diagnosis.

7. The lesion diagnosis apparatus of claim 4, wherein the at least one processor is further configured to exclude at least one lesion that has been recognized by the user, from an area in which lesions are to be detected.

8. A method of providing guiding information, comprising:
detecting, with at least one processor of a lesion diagnosis apparatus, at least one lesion in images that have been received from an ultrasound probe movable on the surface of a body part of a patient;
storing location information of the at least one detected lesion in a memory;
displaying the images received from the ultrasound probe;
generating, by the at least one processor, a detection signal to notify a user that the at least one lesion is detected; and
displaying guide information based on the stored location information of the at least one detected lesion when the lesion has been detected and in response to a movement of the ultrasound probe leaving a location of the detected lesion,
wherein the guide information is a visualized image for guiding the user to move the ultrasound probe toward the location of the detected lesion, and the guide information comprises at least one of a moving path, a moving distance, a moving direction, or a measured angle of the ultrasound probe.

9. The method of claim 8, further comprising storing lesion information when the lesion is detected, and the lesion information comprises at least one of features of the lesion, diagnosis of the lesion, location information of the lesion, or information about the movement of the ultrasound probe after the lesion is detected.

10. The method of claim 8 or 9, wherein the detecting of the at least one lesion is performed in real-time and comprises:
detecting at least one lesion in a first image received from the ultrasound probe;
identifying a non-overlapping area between the first image and a second image received from the ultrasound probe; and
detecting at least one lesion only in the non-overlapping area of the second image.

11. The method of claim 10, wherein the non-overlapping area is identified using at least one of coordinates, a measuring range, a measured angle, or a measurement direction of the ultrasound probe.

12. The method of claim 10, further comprising:
receiving a confirmation from the user that the detected lesion is recognized, and
wherein the providing of the guide information comprises continuing to provide the guide information until the user confirms that the detected lesion is recognized.

13. Computer program product, comprising one or more computer readable media having computer-executable instructions for performing the steps of the method according to one of the Claims 8 - 12.

## Patentansprüche

1. Läsionsdiagnosevorrichtung, die Folgendes umfasst:
einen Speicher, der zum Speichern von Anweisungen konfiguriert ist; und
wenigstens einen Prozessor, der bei Ausführung der gespeicherten Anweisungen konfiguriert ist zum:
Erfassen wenigstens einer Läsion in empfangenen, von einer auf der Oberfläche eines Körperteils eines Patienten beweglichen Ultraschallsonde stammenden Bildern;
Anzeigen der empfangenen, von der Ultraschallsonde stammenden Bilder; und
Informieren eines Benutzers, dass die wenigstens eine Läsion erfasst wurde,
**DADURCH GEKENNZEICHNET, DASS**
der wenigstens eine Prozessor weiterhin konfiguriert ist zum:
Steuern des Speichers dazu, Positionsinformationen über die wenigstens eine erfasste Läsion zu speichern;
Anzeigen von Führungsinformationen basierend auf den gespeicherten Positionsinformationen über die wenigstens eine erfasste Läsion , wenn die Läsion erfasst wurde, und als Reaktion auf eine Bewegung der Ultraschallsonde, die eine Position der erfassten Läsion verlässt, wobei es sich bei den Führungsinformationen um ein visualisiertes Bild zum Führen des Benutzers, um die Ultraschallsonde zu der Position der erfassten Läsion zu bewegen, handelt, und wobei die Führungsinformationen einen Bewegungspfad, eine Bewegungsentfernung, eine Bewegungsrichtung und/oder einen gemessenen Winkel der Ultraschallsonde umfassen.

2. Läsionsdiagnosevorrichtung nach Anspruch 1, wobei der wenigstens eine Prozessor weiterhin konfiguriert ist zum:
Erfassen von wenigstens einer Läsion in einem ersten empfangenen, von der Ultraschallsonde stammenden Bild;
Identifizieren eines nicht überlappenden Bereichs zwischen dem ersten Bild und einem zweiten empfangenen, von der Ultraschallsonde stammenden Bild; und
Erfassen von wenigstens einer Läsion nur in dem nicht überlappenden Bereich des zweiten Bildes.

3. Läsionsdiagnosevorrichtung nach Anspruch 1 oder 2, wobei der Speicher zum Speichern von Läsionsinformationen konfiguriert ist, wenn die Läsion erfasst wird, und die Läsionsinformationen wenigstens eines der Folgenden umfassen: Merkmale der Läsion, eine Diagnose der Läsion, Positionsinformationen der Läsion und Informationen über die Bewegung der Ultraschallsonde, nachdem die Läsion erfasst wurde; und
wobei die Merkmale der Läsion wenigstens eines der Folgenden umfassen: die Form der Läsion, die Grenze der Läsion, einen hinteren akustischen Schatten der Läsion, ein Echo-Muster, Mikrolobulationen, einen hypoechoischen Halo, einen hypoechoischen Knoten, eine Kanalverlängerung, die Kompressibilität, das Verzweigungsmuster und die Dichte der Läsionen.

4. Läsionsdiagnosevorrichtung nach Anspruch 3, die weiterhin Folgendes umfasst:
eine Eingabevorrichtung, die dazu konfiguriert ist, eine Bestätigung von dem Benutzer zu empfangen, dass die erfasste Läsion erkannt wird; und
wobei die Läsionsinformationen weiterhin eine Anzeige umfassen, ob der Benutzer die erfasste Läsion erkannt hat.

5. Läsionsdiagnosevorrichtung nach Anspruch 4, wobei der wenigstens eine Prozessor dazu konfiguriert ist, ein Erfassungssignal zu erzeugen, wenn wenigstens eine Läsion, die von dem Benutzer nicht erkannt wurde, erneut erfasst wird.

6. Läsionsdiagnosevorrichtung nach Anspruch 4, wobei der wenigstens eine Prozessor dazu konfiguriert ist, ein Erfassungssignal zu erzeugen, wenn wenigstens eine Läsion erneut erfasst wird, deren Diagnose sich von einer vorherigen Diagnose unterscheidet.

7. Läsionsdiagnosevorrichtung nach Anspruch 4, wobei der wenigstens eine Prozessor dazu konfiguriert ist, die wenigstens eine Läsion, die von dem Benutzer erkannt wurde, aus einem Bereich, in dem Läsionen erfasst werden sollen, auszuschließen.

8. Verfahren zur Bereitstellung von Führungsinformationen, wobei das Verfahren Folgendes umfasst:
Erfassen, mit Hilfe wenigstens eines Prozessors einer Läsionsdiagnosevorrichtung, wenigstens einer Läsion in empfangenen, von einer auf der Oberfläche eines Körperteils eines Patienten beweglichen Ultraschallsonde stammenden Bildern;
Speichern von Positionsinformationen über die wenigstens eine erfasste Läsion in einem Speicher;
Anzeigen der empfangenen, von der Ultraschallsonde stammenden Bilder;
Erzeugen, durch den wenigstens einen Prozessor, eines Erfassungssignals zum Informieren eines Benutzers, dass die wenigstens eine Läsion erfasst wurde; und
Anzeigen von Führungsinformationen basierend auf den gespeicherten Positionsinformationen über die wenigstens eine erfasste Läsion, wenn die Läsion erfasst wurde, und als Reaktion auf eine Bewegung der Ultraschallsonde, die eine Position der erfassten Läsion verlässt,
wobei es sich bei den Führungsinformationen um ein visualisiertes Bild zum Führen des Benutzers, um die Ultraschallsonde zu der Position der erfassten Läsion zu bewegen, handelt, und wobei die Führungsinformationen einen Bewegungspfad, eine Bewegungsentfernung, eine Bewegungsrichtung und/oder einen gemessenen Winkel der Ultraschallsonde umfassen.

9. Verfahren nach Anspruch 8, weiterhin umfassend das Speichern von Läsionsinformationen, wenn die Läsion erfasst wird, wobei die Läsionsinformationen wenigstens eines der Folgenden umfassen: Merkmale der Läsion, eine Diagnose der Läsion, Positionsinformationen der Läsion und Informationen über die Bewegung der Ultraschallsonde, nachdem die Läsion erfasst wurde.

10. Verfahren nach Anspruch 8 oder 9, wobei das Erfassen der wenigstens einen Läsion in Echtzeit durchgeführt wird und Folgendes umfasst:
Erfassen von wenigstens einer Läsion in einem ersten empfangenen, von der Ultraschallsonde stammenden Bild;
Identifizieren eines nicht überlappenden Bereichs zwischen dem ersten Bild und einem zweiten empfangenen, von der Ultraschallsonde stammenden Bild; und
Erfassen von wenigstens einer Läsion nur in dem nicht überlappenden Bereich des zweiten Bildes.

11. Verfahren nach Anspruch 10, wobei der nicht überlappende Bereich unter Verwendung von wenigstens einem der Folgenden identifiziert wird: Koordinaten, einem Messbereich, einem gemessenen Winkel und einer Messrichtung der Ultraschallsonde.

12. Verfahren nach Anspruch 10, das weiterhin Folgendes umfasst:
Empfangen einer Bestätigung von dem Benutzer, dass die erfasste Läsion erkannt wird; und
wobei das Bereitstellen der Führungsinformationen ein durchgehendes Bereitstellen der Führungsinformationen umfasst, bis der Benutzer bestätigt, dass die erfasste Läsion erkannt wird.

13. Computerprogrammprodukt, umfassend ein oder mehrere computerlesbare Medien mit durch einen Computer ausführbaren Anweisungen zur Durchführung der Schritte des Verfahrens nach einem der Ansprüche 8 bis 12.

## Revendications

1. Dispositif de diagnostic de lésion comprenant :
une mémoire conçue pour stocker des instructions, et
au moins un processeur qui, lors de l'exécution des instructions stockées, est conçu pour :
détecter au moins une lésion dans des images reçues en provenance d'une sonde à ultrasons déplaçable sur la surface d'une partie du corps d'un patient,
afficher les images reçues en provenance de la sonde à ultrasons, et
avertir un utilisateur que l'au moins une lésion est détectée,
**CARACTÉRISÉ EN CE QUE**
l'au moins un processeur est conçu en outre pour :
commander la mémoire afin d'y stocker des informations d'emplacement concernant l'au moins une lésion détectée,
afficher des informations de guidage en fonction des informations d'emplacement stockées concernant l'au moins une lésion détectée, lorsque la lésion a été détectée et en cas de déplacement de la sonde à ultrasons l'amenant à quitter l'emplacement de la lésion détectée ; lesdites informations de guidage consistant en une image de visualisation permettant de guider l'utilisateur dans le déplacement de la sonde à ultrasons vers l'emplacement de la lésion détectée, et les informations de guidage comprenant au moins un élément parmi : la trajectoire de déplacement, la distance de déplacement, la direction de déplacement et la mesure de l'angle de la sonde à ultrasons.

2. Dispositif de diagnostic de lésion selon la revendication 1, dans lequel l'au moins un processeur est conçu en outre pour :
détecter au moins une lésion dans une première image reçue en provenance de la sonde à ultrasons,
identifier une zone de non chevauchement entre la première image et une deuxième image reçue en provenance de la sonde à ultrasons, et
détecter au moins une lésion uniquement dans la zone de non chevauchement de la deuxième image.

3. Dispositif de diagnostic de lésion selon la revendication 1 ou 2, dans lequel la mémoire est conçue pour stocker des informations de lésion lorsque la lésion est détectée, et les informations de lésion comprennent au moins un élément parmi : des caractéristiques de la lésion, un diagnostic de la lésion, des informations d'emplacement de la lésion et des informations concernant le déplacement de la sonde à ultrasons après que la lésion a été détectée ; et
dans lequel les caractéristiques de la lésion comprennent au moins un élément parmi : la forme de la lésion, le contour de la lésion, une ombre acoustique postérieure de la lésion, l'échostructure, des microlobulations, un halo hyperéchoïque, un nodule hypoéchoïque, une extension ductale, la compressibilité, l'aspect ramifié et la densité des lésions.

4. Dispositif de diagnostic de lésion selon la revendication 3, comprenant en outre :
un moyen d'entrée conçu pour recevoir une confirmation de la part de l'utilisateur que la lésion détectée a été reconnue, et
les informations de lésion comprennent en outre une indication portant sur le fait que l'utilisateur a reconnu la lésion détectée.

5. Dispositif de diagnostic de lésion selon la revendication 4, dans lequel l'au moins un processeur est conçu pour produire un signal de détection lorsqu'au moins une lésion qui n'a pas été reconnue par l'utilisateur est détectée de nouveau.

6. Dispositif de diagnostic de lésion selon la revendication 4, dans lequel l'au moins un processeur est conçu pour produire un signal de détection lorsqu'au moins une lésion est détectée de nouveau et que son diagnostic est différent d'un diagnostic précédent.

7. Dispositif de diagnostic de lésion selon la revendication 4, dans lequel l'au moins un processeur est conçu en outre pour exclure d'une zone, dans laquelle des lésions doivent être détectées, l'au moins une lésion qui a été reconnue par l'utilisateur.

8. Procédé visant à fournir des informations de guidage, comprenant :
la détection, au moyen d'au moins un processeur d'un dispositif de diagnostic de lésion, d'au moins une lésion sur des images reçues en provenance d'une sonde à ultrasons déplaçable sur la surface d'une partie du corps d'un patient,
le stockage, dans une mémoire, d'informations d'emplacement concernant l'au moins une lésion détectée,
l'affichage des images reçues en provenance de la sonde à ultrasons,
la production, par l'au moins un processeur, d'un signal de détection visant à avertir un utilisateur que l'au moins une lésion a été détectée, et
l'affichage d'informations de guidage en fonction des informations d'emplacement stockées concernant l'au moins une lésion détectée, lorsque la lésion a été détectée et en cas de déplacement de la sonde à ultrasons l'amenant à quitter l'emplacement de la lésion détectée,
les informations de guidage consistant en une image de visualisation permettant de guider l'utilisateur dans le déplacement de la sonde à ultrasons vers l'emplacement de la lésion détectée, et les informations de guidage comprenant au moins un élément parmi : la trajectoire de déplacement, la distance de déplacement, la direction de déplacement et la mesure de l'angle de la sonde à ultrasons.

9. Procédé selon la revendication 8, comprenant en outre le stockage d'informations de lésion lorsque la lésion est détectée, les informations de lésion comprenant au moins un élément parmi : des caractéristiques de la lésion, un diagnostic de la lésion, des informations d'emplacement de la lésion et des informations concernant le déplacement de la sonde à ultrasons après que la lésion a été détectée.

10. Procédé selon la revendication 8 ou 9, dans lequel la détection de l'au moins une lésion s'effectue en temps réel et comprend :
la détection d'au moins une lésion dans une première image reçue en provenance de la sonde à ultrasons,
l'identification d'une zone de non chevauchement entre la première image et une deuxième image reçue en provenance de la sonde à ultrasons, et
la détection d'au moins une lésion uniquement dans la zone de non chevauchement de la deuxième image.

11. Procédé selon la revendication 10, dans lequel la zone de non chevauchement est identifiée au moyen d'au moins un élément parmi : des coordonnées, une plage de mesure, une mesure d'angle et une direction de mesure de la sonde à ultrasons.

12. Procédé selon la revendication 10, comprenant en outre :
la réception d'une confirmation, en provenance de l'utilisateur, que la lésion détectée est reconnue ;
la fourniture des informations de guidage comprenant la continuation de la fourniture des informations de guidage jusqu'à ce que l'utilisateur confirme que la lésion détectée est reconnue.

13. Produit-programme informatique, comprenant un ou plusieurs supports lisibles par ordinateur portant des instructions exécutables par ordinateur pour réaliser les étapes du procédé selon l'une quelconque des revendications 8 à 12.
